(19) Europäisches Patentamt / European Patent Office / Office européen des brevets

(11) **EP 3 509 551 B1**

(12) **EUROPEAN PATENT SPECIFICATION**

(45) Date of publication and mention
of the grant of the patent:
**10.12.2025 Bulletin 2025/50**

(21) Application number: **17758004.0**

(22) Date of filing: **16.08.2017**

(51) International Patent Classification (IPC):
***A61F 13/49*** *(2006.01)*

(52) Cooperative Patent Classification (CPC):
**A61F 13/4902**

(86) International application number:
**PCT/US2017/047056**

(87) International publication number:
**WO 2018/048590 (15.03.2018 Gazette 2018/11)**

(54) **STRETCH NONWOVENS AND FILMS**

ELASTISCHE VLIESSTOFFE UND FOLIEN

FILMS ET NON-TISSÉS ÉTIRABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.09.2016 US 201662384217 P**

(43) Date of publication of application:
**17.07.2019 Bulletin 2019/29**

(73) Proprietor: **The LYCRA Company UK Limited
Manchester
M2 3DE (GB)**

(72) Inventors:
• **BISSAH, Kofi
  Newark, Delaware 19702 (US)**
• **GILBERT, Michael
  Bear, Delaware 19701 (US)**

(74) Representative: **Carpmaels & Ransford LLP
One Southampton Row
London WC1B 5HA (GB)**

(56) References cited:
WO-A1-2009/127966  WO-A1-2015/084865
US-A- 4 366 814  US-A1- 2006 143 767
US-A1- 2006 183 849  US-A1- 2011 041 232

## Description

### FIELD OF INVENTION

The present invention relates to elastic polymer compositions that provide stretch recovery to absorbent fabrics and products produced from these absorbent fabrics. Further provided are thin films that possess stretch recovery without tackiness. These compositions are particularly useful in providing stretch recovery by dispersion printing on fabrics used in disposable personal care items.

### SUMMARY OF RELATED TECHNOLOGY

Polymer compositions such as polyurethaneurea films and tapes that provide stretch recovery are disclosed in U.S. Patent No. 7,240,371.

Carmen C. et al disclosed a method to add polymer composition on the edge of garments to form the garment edge bands and to add film on garments such as brassiere to form laminate fabrics in patent EP 2280619B1 and US2009/0181599A1.

WO2015084865 discloses a shape enhancing garment including a base elastic fabric region and at least one fabric composite zone, wherein, an elastic polymer composition, such as a polyurethaneurea, a polyurethane, or a polyolefin, is discontinuously placed in the fabric back, penetrates and anchors into the fabric inside, and is not visible from the outside of the fabric.

US2011/041232A1 discloses garments that have at least one opening and include a polymer composition which provides benefits to the garment opening including stretch recovery/elasticity and shape retention.

### SUMMARY OF THE INVENTION

Disposable personal care items with stretch recovery are highly desirable.

One aspect of the present invention relates to the use of an elastic polymer composition for providing stretch recovery to an absorbent fabric or a product produced from an absorbent fabric, wherein the elastic polymer composition is a polymer coated stretch film, and wherein said elastic polymer composition is dispersion printed onto the absorbent fabric, wherein the absorbent fabric is used in a disposable personal care item.

In one nonlimiting embodiment, the elastic polymer composition exhibits elongation from about 5 to about 2000%.

In one nonlimiting embodiment, the polymer coating comprises polyurethane, latex or rubber solution, and/or styrene block co-polymer.

Another aspect of the present invention relates to a method for enhancing stretch recovery of an absorbent fabric. In this method, an elastic polymer composition is dispersion printed on the absorbent fabric, wherein the elastic polymer composition is a polymer coated stretch film, and wherein the absorbent fabric is used in a disposable personal care item.

In one nonlimiting embodiment, the elastic polymer composition exhibits elongation from about 5 to about 2000%.

In one nonlimiting embodiment, the polymer coating comprises polyurethane, latex or rubber solution, and/or styrene block co-polymer.

In one nonlimiting embodiment, the disposable person care item is a diaper, a feminine hygiene product or an incontinence pad.

### DETAILED DESCRIPTION OF THE INVENTION

Disclosed are elastic polymer compositions that provide stretch recovery to absorbent fabrics and products produced from these absorbent fabrics. These compositions are particularly useful in providing stretch recovery by dispersion printing on fabrics used in disposable personal care items including, but not limited to, diapers, feminine hygiene products and incontinence pads.

As used herein, the term "film" means a flat, generally two-dimensional article. The film may be self-supporting such as a film that has been cast and dried or extruded. Alternatively, the film may be a melt, dispersion or solution.

As used herein, the term "dispersion" refers to a system in which the dispersed phase consists of finely divided particles, and the continuous phase can be a liquid, solid or gas.

As used herein, the term "aqueous polyurethane dispersion" refers to a composition containing at least a polyurethane or polyurethane urea polymer or prepolymer (such as the polyurethane prepolymer described herein), optionally including a solvent, that has been dispersed in an aqueous medium, such as water, including de-ionized water.

As used herein, the term "solvent," unless otherwise indicated, refers to a non-aqueous medium, wherein the non-aqueous medium includes organic solvents, including volatile organic solvents (such as acetone) and somewhat less volatile organic solvents (such as MEK, or NMP). As used herein, the term "solvent-free" or "solvent-free system" refers to a composition or dispersion wherein the bulk of the composition or dispersed components has not been dissolved or dispersed in a solvent.

As used herein, the term "fabric" refers to a knitted, woven or nonwoven material. The knitted fabric may be flat knit, circular knit, warp knit, narrow elastic, and lace. The woven fabric may be of any construction, for example sateen, twill, plain weave, oxford weave, basket weave, and narrow elastic. The nonwoven material may be melt blown, spun bonded, wet-laid, carded fiber-based staple webs, and the like. By "fabric" it is meant to be inclusive of absorbent fabrics.

As used herein, the term "derived from" refers to forming a substance out of another object. For example, a film may be derived from a dispersion which can be dried.

Disposable personal care items of some aspects are advantageously constructed with areas of fabric composite at specific locations to provide stretch recovery. As used herein, the term 'fabric composite' preferably comprises, for example, elastic polymer composition applied to an absorbent fabric, which is both absorbent and exhibits stretch recovery. Without being bound to any particular theory, it is believed that polymer particles of the elastic polymer composition discontinuously locate and stick with fibers and yarns, and separately penetrate into the fabric body.

Elastomeric fibers are commonly used to provide stretch and elastic recovery in fabrics and garments. "Elastomeric fibers" are either a continuous filament (optionally a coalesced multifilament) or a plurality of filaments, free of diluents, which have a break elongation in excess of 100% independent of any crimp. An elastomeric fiber when (1) stretched to twice its length; (2) held for one minute; and (3) released, retracts to less than 1.5 times its original length within one minute of being released. As used in the text of this specification, "elastomeric fibers" means at least one elastomeric fiber or filament. Such elastomeric fibers include but are not limited to rubber filament, biconstituent filament (which may be based on rubber, polyurethane, etc.), lastol, and spandex. The terms "elastomeric" and "elastic" are used interchangeably throughout the specification.

"Spandex" is a manufactured filament in which the filament-forming substance is a long chain synthetic polymer comprised of at least 85% by weight of segmented polyurethane. "Elastoester" is a manufactured filament in which the fiber forming substance is a long chain synthetic polymer composed of at least 50% by weight of aliphatic polyether and at least 35% by weight of polyester. Although not elastomeric, elastoester may be included in some fabrics herein.

"Polyester bi-component filament" means a continuous filament comprising a pair of polyesters intimately adhered to each other along the length of the fiber, so that the fiber cross section is for example a side-by-side, eccentric sheath-core or other suitable cross-section from which useful crimp can be developed. The polyester bicomponent filament comprises poly(trimethylene terephthalate) and at least one polymer selected from the group consisting of poly(ethylene terephthalate), poly(trimethylene terephthalate), and poly(tetramethylene terephthalate) or a combination of such members, having an after heat-set crimp contraction value of from about 10% to about 80%.

In accordance with the present invention the elastic polymer composition is a polymer coated stretch film.

Examples of films include, but are not limited to, single solutions polyurethanes, derivatives of polyurethanes, latex NRL, silicones and other compositions that are cured. Thickness of the film ranges from about 0.5 mil to about 5 mil.

Examples of polymer coatings useful in the elastic polymer compositions include, but are not limited to, polyurethane, latex or rubber solutions, and styrene block co-polymers and combinations or blends thereof.

Elastic polymer compositions of the present invention exhibit elongation from about 5 to about 2000%. In one nonlimiting embodiment, when the elastic polymer composition is a polymer coated stretch nonwoven, it exhibits an elongation from about 5% to about 200%. In an alternative nonlimiting embodiment, when the elastic polymer composition is a polymer coated film, it exhibits an elongation from about 400% to about 2000%.

The elastic polymer compositions of the present invention can be applied to absorbent fabrics to provide stretch recovery. Unlike film or fabric laminate in prior arts, within the innovation fabric, the polymer composition does not form a film or a continuous flat surface. When dispersion is used, the divided polymers particles are discontinuously placed and separately penetrate into the fabric body thereby avoiding any unpleasant shiny and/or rubbery touch surface. The elastic polymer composition is also invisible from the outside of any products produced from the composition and the products exhibit good breathability.

The elastic polymer compositions can be dispersed by a variety of methods including, but not limited to, heat/bonding, spreading, painting, brushing, spraying, kissing, printing, dipping, padding, dispensing, metering, and combinations thereof. This may be followed by application of heat and/or pressure.

In one nonlimiting embodiment, the elastic polymer composition is dispersion printed onto a fabric or onto a release paper. The dispersion can be printed by various print methods including: overlay screen printing and gravure printing. The print design can be engineered to impart desired attributes to products such as: directional strength, support, uni directional stretch, bi-directional stretch, stretch and receovery, fluid transfer efficiency or imcreased absorbancy.

The base fabric itself can be any absorbent fabric used in production of disposable personal items such as, but not limited to, diapers, feminine hygiene products and incontinence pads. The inclusion of the polyurethaneurea composition imparts benefits of stretch recovery to the absorbent fabric. They can be used in a variety of different disposable personal items such as, but not limited to, diapers, feminine hygiene products and incontinence pads, wipes, nursing pads, breast pads, dusting pads, mattress pads, wound pads,

A disposable personal care item with stretch recovery is provided by applying the elastic polymer compositions in divided particles form in targeted areas. The elastic polymer composition may be applied to the absorbent fabric prior to preparation of the disposable personal care item, to the disposable personal care item or to both the fabric and disposable personal care item. The elastic polymer composition content is about 1% and to about 30% of base fabric weight.

Other additives that may be optionally included in the elastic polymer composition include, but are not limited to, antioxidants, UV stabilizers, colorants, pigments, crosslinking agents, phase change materials (i.e., Outlast®, commercially available from Outlast Technologies, Boulder, Colorado), antimicrobials, minerals (i.e., copper), microencapsulated wellbeing additives (i.e., aloe vera, vitamin E gel, aloe vera, sea kelp, nicotine, caffeine, scents or aromas), nanoparticles (i.e., silica or carbon), calcium carbonate, flame retardants, antitack additives, chlorine degradation resistant additives, vitamins, medicines, fragrances, electrically conductive additives, and/or dye-assist agents (i.e., Methacrol®, commercially available from E. I. DuPont de Nemours, Wilmington, Delaware). Other additives which may be added to the elastic polymer composition comprise adhesion promoters, antistatic agents, anti-cratering agents, anti-crawling agents, optical brighteners, coalescing agents, electroconductive additives, luminescent additives, flow and leveling agents, freeze-thaw stabilizers, lubricants, organic and inorganic fillers, preservatives, texturizing agents, thermochromic additives, insect repellants, and wetting agents. Such optional additives may be added to the elastic polymer composition before, during, or after the elastic polymer composition is dispersed, as the process allows.

At least one coagulant may optionally be used to control the penetration of dispersions into a fabric or other article. Examples of coagulants that may be used include calcium nitrate (including calcium nitrate tetrahydrate), calcium chloride, aluminum sulfate (hydrated), magnesium acetate, zinc chloride (hydrated) and zinc nitrate.

The coating, dispersion, or fabric may be pigmented or colored and also may be used as a design element in that regard.

Examples of disposable personal items that can be produced using the elastic polymer compositions falling within the scope of the present invention, include but are not limited to: diapers, feminine hygiene products, incontinence pads, etc.

**ANALYTICAL METHOD**

In the examples that follow, the following analytical methods were used.

**Fabric Elongation (Stretch)**

Fabrics are evaluated for % elongation under a specified load (i.e., force) in the fabric stretch direction(s), which is the direction of the composite yarns (i.e., weft, warp, or weft and warp). Three samples of dimensions 20 cm x 6.5 cm were cut from the fabric. The long dimension (25 cm) corresponds to the stretch direction. The samples are partially unraveled to reduce the sample widths to 5.0 cm. The samples are then conditioned for at least 16 hours at 20°C +/- 2°C and 65% relatively humidity, +/- 2%.

A first benchmark was made across the width of each sample, at 6.5 cm from a sample end. A second benchmark was made across the sample width at 20.0 cm from the first benchmark. The excess fabric from the second benchmark to the other end of the sample was used to form and stitch a loop into which a metal pin could be inserted. A notch was then cut into the loop so that weights could be attached to the metal pin.

The sample non-loop end was clamped and the fabric sample was hung vertically. A 17.8 Newton (N) weight (4 LB) is attached to the metal pin through the hanging fabric loop, so that the fabric sample is stretched by the weight. The sample was "exercised" by allowing it to be stretched by the weight for three seconds, and then manually relieving the force by lifting the weight. This cycle was carried out three times. The weight was allowed then to hang freely, thus stretching the fabric sample. The distance in millimeters between the two benchmarks was measured while the fabric was under load, and this distance is designated ML. The original distance between benchmarks (i.e., unstretched distance) was designated GL. The % fabric elongation for each individual sample as calculated as follows:

$$\% \text{ Elongation (E\%)} = ((ML\text{-}GL)/GL) \times 100$$

The three elongation results were averaged for the final result. The following section provides further illustration of the compositions of the present invention. These working examples are illustrative only and are not intended to limit the scope of the invention in any way.

| Elongation @ Break | Ld @ 10% | Ld @ 20% | Ld @ 30% | Ld @ 50% | Ld @ 60% | Load @ 100% | Load @ 200% | Load @ 300% | Ld @ 400% | Ld @ 500% | Ld @ 550% |
|---|---|---|---|---|---|---|---|---|---|---|---|
| (%) | (gf) | (gf) | (gf) | (gf) | (gf) | (gf) | (gf) | (gf) | (gf) | (gf) | (gf) |
| 1829.2 | 10.03 | 19.72 | 29.37 | 47.21 | 54.89 | 78.51 | 111.48 | 130.04 | 143.45 | 154.38 | 159.85 |
| 1831.5 | 8.91 | 18.12 | 28.26 | 46.58 | 54.5 | 79.74 | 117.13 | 138.06 | 152.8 | 165.35 | 171.31 |
| 1836 | 14.67 | 22.32 | 30.51 | 45.86 | 53.45 | 78.12 | 116.19 | 137.73 | 153.2 | 165.74 | 171.89 |
| **1832.2** | **11.21** | **20.06** | **29.38** | **46.55** | **54.28** | **78.79** | **114.94** | **135.28** | **149.82** | **161.82** | **167.68** |

**EXAMPLES**

Nonwovens are commercially available materials including "spunbond fabrics" spun lace non-woven fabric, airlaid nonwoven webs and materials, wetlaid nonwoven fabrics and meltspun fiber containing fabrics and materials, and are mostly comprised of polyesters (such as PET or PBT), polyolefins (including PP and PE), PP/PE Bicomponent fibers, or PET/PP Bicomponent fiber with basis weight ranging from 20gsm to 200gsm. Basis weight (Z) is determined by dividing the weight of nonwoven (Xgrams) by the unit area (Ysm(square meters). Z= X/Y [Zgsm]

A Stretch nonwoven material is achieved by coating the nonwoven with a polyurethane dispersion (PUD), drying by force air or at ambient and curing at high temperature (100C- 200C) and at residence time (5secs- 45secs) to impart elastic properties to the nonwoven. Several techniques are available for the solution coating, however, for this invention, screen printing, kiss coating, rotogravure coating, offset printing are among the preferred modes. Employing screen patterns allows for selective printing thus imparting surface patterns to the coated nonwoven. For example you can opt for a screen design where there are opened areas and closed areas. The PUD coating will migrate through the open areas of the screen to coat the nonwoven resulting in selective coating or pattern coating.

**Example 1: Preparation and Testing of Stretch Nonwoven at the Laboratory level**

1. An extensible nonwoven preferably transverse extensibility is die cut to size: 8inches wide and 11 inches long.
2. Determine the weight of nonwoven specimen in grams [A]
3. Determine the area in meters squared [B]
4. Place a screen( all open screen or selective screen) on the dry nonwoven
5. Pour PUD on to the screen and using screen printing blade print the PUD via the screen opening onto the nonwoven.
6. Dry the wet coated nonwoven by force air or ambient
7. Cure the dried nonwoven by heat and residence time
8. Determine the weight of the cured nonwoven specimen in grams [C]

$$\text{Basis weight of nonwoven before coating} = A/B$$

$$\text{Basis weight of (Stretch nonwoven) coated nonwoven or cured nonwoven} = C/B$$

Using traditional INSTRON tensile methodology the Elongation of the stretch nonwoven was determined to be in the range 5-200%. With allowing stretch recovery 100% to 50%. Thus the Set range 0- 50%. 0% set means 100% recovery; 50% set means 50% recovery.

The moisture vapor transmission rate (MVTR) of this stretch woven sample ranged from 100 - 4000 g/m$^2$ over 24 hours.

**Example 2: Preparation and Testing of Stretch Film**

A polyurethane solution coated film (PUD) was prepared as follows. The thickness of the film can range from 0.5 mil to 5 mil.

Elongation of the stretch film was determined to be 400 - 2000%. The moisture vapor transmission rate (MVTR) of this stretch film sample ranged from 50 - 2600g/m$^2$ over 24 hours.

**Example 3: MVTR of Breathable Stretch Film**

The analytical method used for determination of MVTR Moisture vapor transmission rate as ASTM E96.

| Moisture Vapor Transmission Rate : A measure of breathability | | | |
|---|---|---|---|
| Sample | Description | Manufacturer | MVTR gms/24 Hrs/sq.m |
| 0 | Innovative sample - breathable film | INVISTA | 2588 |
| | | | |

(continued)

| | | Adult incontinence | | |
|---|---|---|---|---|
| | 1 | Always Discreet Belly | P&G | 1453.6 |
| | 2 | Depend for women Fit Flex Belly | KC | 1725.36 |
| | 3 | Prevail with odorguard Belly | FQ | 1226.08 |
| | 4 | Tena underwear Belly | SCA | 756.82 |
| | 5 | Depend Silhouette Active Fit Belly | KC | 1038.06 |
| | | | | |
| | | Diapers and Training pants | | |
| | 6 | Huggies little swimmers side panels (stretched) | KC | 2155.12 |
| | 7 | Boys training pants Up&Up side panels (stretched) | FQ | 2216.74 |
| | 8 | Huggies Litte Movers side panel (stretched) | KC | **112.18** |
| | 9 | Huggies Pull-ups training pants side panel (stretched) | KC | 1883.36 |
| | 10 | Pampers Easy Ups training pants side panels (stretched) | P&G | 1139.18 |
| | 11 | Pampers Splashers side panels (stretched) | P&G | 1142.34 |
| | 12 | Goodnite Bedtime underwear side panels (stretched) | KC | 1881.78 |
| | 13 | CVS I'm on the Move side panel (stretched) | AHP Domtar | 1532.6 |
| | 14 | Luvs Ultra Leak Guards side panels (stretched) | P&G | **52.14** |
| | 15 | Huggie little movers training pants (stretched) | KC | 2102.98 |

## Claims

1. Use of an elastic polymer composition for providing stretch recovery to an absorbent fabric or a product produced from an absorbent fabric, said elastic polymer composition is a polymer coated stretch film, wherein the film is a melt, dispersion or solution, and wherein said elastic polymer composition is dispersion printed onto the absorbent fabric, wherein the absorbent fabric is used in a disposable personal care item.

2. The use of claim 1, wherein the polymer coating comprises polyurethane, latex or rubber solution, and/or styrene block co-polymer.

3. The use of claim 1, wherein the elastic polymer composition exhibits elongation from about 5% to about 2000%, wherein elongation is measured under a force of 17.8 Newton in the stretch direction according to the procedure on pages 7 and 8 of the specification.

4. The use of claim 1, wherein the elastic polymer composition exhibits elongation from about 5% to about 200%, wherein elongation is measured under a force of 17.8 Newton in the stretch direction according to the procedure on pages 7 and 8 of the specification.

5. The use of claim 1, wherein the elastic polymer composition exhibits elongation from about 200% about 400%, wherein elongation is measured under a force of 17.8 Newton in the stretch direction according to the procedure on pages 7 and 8 of the specification.

6. The use of claim 1, wherein the elastic polymer composition exhibits elongation from about 400% to about 2000%, wherein elongation is measured under a force of 17.8 Newton in the stretch direction according to the procedure on pages 7 and 8 of the specification.

7. A method for enhancing stretch recovery of an absorbent fabric, said method comprising dispersion printing the elastic polymer composition defined in any of claims 1-6 on the absorbent fabric, wherein the absorbent fabric is used in a disposable personal care item.

8. The use of claim 1 or method of claim 7 wherein the disposable personal care item is a diaper, a feminine hygiene product, an incontinence pad, nursing pad, wipes, breast pads, mattress pads, dusting pads, or facial mask.

**Patentansprüche**

1. Verwendung einer elastischen Polymerzusammensetzung zum Bereitstellen einer Dehnungsrückgewinnung für ein saugfähiges Gewebe oder ein Produkt, das aus einem saugfähigen Gewebe hergestellt wird, wobei die elastische Polymerzusammensetzung eine polymerbeschichtete Stretchfolie ist, wobei die Folie eine Schmelze, Dispersion oder Lösung ist, und wobei die elastische Polymerzusammensetzung eine Dispersion ist, die auf das saugfähige Gewebe gedruckt ist, wobei das saugfähige Gewebe in einem Einweg-Körperpflegeartikel verwendet wird.

2. Verwendung nach Anspruch 1, wobei die Polymerbeschichtung Polyurethan-, Latex- oder Gummilösung und/oder Styrol-Blockcopolymer umfasst.

3. Verwendung nach Anspruch 1, wobei die elastische Polymerzusammensetzung eine Dehnung von etwa 5 % bis etwa 2000 % aufweist, wobei die Dehnung unter einer Kraft von 17,8 Newton in der Dehnungsrichtung gemäß dem Verfahren auf den Seiten 7 und 8 der Spezifikation gemessen wird.

4. Verwendung nach Anspruch 1, wobei die elastische Polymerzusammensetzung eine Dehnung von etwa 5 % bis etwa 200 % aufweist, wobei die Dehnung unter einer Kraft von 17,8 Newton in der Dehnungsrichtung gemäß dem Verfahren auf den Seiten 7 und 8 der Spezifikation gemessen wird.

5. Verwendung nach Anspruch 1, wobei die elastische Polymerzusammensetzung eine Dehnung von etwa 200 % bis etwa 400 % aufweist, wobei die Dehnung unter einer Kraft von 17,8 Newton in der Dehnungsrichtung gemäß dem Verfahren auf den Seiten 7 und 8 der Spezifikation gemessen wird.

6. Verwendung nach Anspruch 1, wobei die elastische Polymerzusammensetzung eine Dehnung von etwa 400 % bis etwa 2000 % aufweist, wobei die Dehnung unter einer Kraft von 17,8 Newton in der Dehnungsrichtung gemäß dem Verfahren auf den Seiten 7 und 8 der Spezifikation gemessen wird.

7. Verfahren zur Verbesserung der Dehnungsrückgewinnung eines saugfähigen Gewebes, wobei das Verfahren das Dispersionsdrucken der elastischen Polymerzusammensetzung, die in einem der Ansprüche 1 bis 6 definiert ist, auf das saugfähige Gewebe umfasst, wobei das saugfähige Gewebe in einem Einweg-Körperpflegeartikel verwendet wird.

8. Verwendung nach Anspruch 1 oder Verfahren nach Anspruch 7, wobei der Einweg-Körperpflegeartikel eine Windel, ein Damenhygieneprodukt, eine Inkontinenzeinlage, eine Stilleinlage, Tücher, Stillpads, Matratzenauflagen, Staubfangtücher oder eine Gesichtsmaske ist.

**Revendications**

1. Utilisation d'une composition polymère élastique pour fournir une récupération d'élasticité à un tissu absorbant ou un produit produit à partir d'un tissu absorbant, ladite composition polymère élastique est un film étirable revêtu de polymère, dans laquelle le film est une masse fondue, une dispersion ou une solution, et dans laquelle ladite composition polymère élastique est imprimée en dispersion sur le tissu absorbant, dans laquelle le tissu absorbant est utilisé dans un article de soins personnels jetable.

2. Utilisation selon la revendication 1, dans laquelle le revêtement polymère comprend du polyuréthane, du latex ou une solution de caoutchouc, et/ou un copolymère à blocs de styrène.

3. Utilisation selon la revendication 1, dans laquelle la composition polymère élastique présente un allongement d'environ 5 % à environ 2 000 %, dans laquelle l'allongement est mesuré sous une force de 17,8 Newton dans la direction d'étirement selon la procédure aux pages 7 et 8 de la spécification.

4. Utilisation selon la revendication 1, dans laquelle la composition polymère élastique présente un allongement d'environ 5 % à environ 200 %, dans laquelle l'allongement est mesuré sous une force de 17,8 Newton dans la direction d'étirement selon la procédure aux pages 7 et 8 de la spécification.

5. Utilisation selon la revendication 1, dans laquelle la composition polymère élastique présente un allongement d'environ 200 % à environ 400 %, dans laquelle l'allongement est mesuré sous une force de 17,8 Newton dans la direction d'étirement selon la procédure aux pages 7 et 8 de la spécification.

6. Utilisation selon la revendication 1, dans laquelle la composition polymère élastique présente un allongement d'environ 400 % à environ 2 000 %, dans laquelle l'allongement est mesuré sous une force de 17,8 Newton dans la direction d'étirement selon la procédure aux pages 7 et 8 de la spécification.

7. Procédé pour améliorer la récupération d'élasticité d'un tissu absorbant, ledit procédé comprenant l'impression en dispersion de la composition de polymère élastique définie dans l'une quelconque des revendications 1 à 6 sur le tissu absorbant, dans lequel le tissu absorbant est utilisé dans un article de soins personnels jetable.

8. Utilisation selon la revendication 1 ou procédé selon la revendication 7 dans laquelle ou lequel l'article de soins personnels jetable est une couche, un produit d'hygiène féminine, une serviette pour incontinent, une compresse d'allaitement, des lingettes, des coussinets mammaires, des couvre-matelas, des tampons de dépoussiérage, ou un masque facial.

**EP 3 509 551 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7240371 B
- EP 2280619 B1
- US 20090181599 A1
- WO 2015084865 A
- US 2011041232 A1